# EUROPEAN PATENT APPLICATION

(11) **EP 4 252 624 A1**
(43) Date of publication of application: **04.10.2023**
(21) Application number: 22165195.3
(22) Date of filing: 29.03.2022
(51) Int. Cl.: A61B 3/10, A61B 3/113

(54) **WEARABLE DEVICES**

(71) Applicant: BIC Violex Single Member S.A., 145 69 Anoixi (GR)
(72) Inventor: Kokozidis, Michail, 14569 Anoixi (GR)
(74) Representative: Peterreins Schley

(57) **Abstract**

The present disclosure relates to a wearable device (100) comprising at least one vibration element (110) configured to vibrate upon receiving an activation signal, wherein the at least one vibration element (110) is configured to be in contact with a user's nose.

## Description

### Technical Field

The present disclosure relates to the field of wearable devices. More specifically, the present disclosure relates to wearable devices comprising vibration elements for tear generation.

### Background

Dry eye syndrome (DES) describes the condition of having dry eyes. Dry eye syndrome occurs when the eye does not produce enough tears or when the tears evaporate too quickly. Insufficient tear production or the quick tear evaporation may lead to inadequate lubrication for the eyes. This can lead to inflammation of the eyes and damage to the eye's surface. Additionally, DES may be permanently uncomfortable to those afflicted. Symptoms include, stinging, burning or scratchy sensations in one's eyes, stringy mucus in or around the eyes, sensitivity to light, eye redness, the sensation of having something in the eyes, difficulty wearing contact lenses, blurred vision and/or eye fatigue.

The prevalence of dry eye syndrome is between about 5-34% depending on the examined population, affecting millions worldwide. The economic impact is estimated to be in the range of billions of dollars.

The treatment usually involves the continuous use of eyedrops, which may be uncomfortable and time-consuming for the user. Further, some affected may require invasive tear duct cautery or intra-nasal electric treatment.

As a result, there is a need for a non-invasive, practicable alternative for reducing the discomfort associated with dry eye syndrome.

### Summary

In a first aspect, the present disclosure relates to a wearable device 100 comprising at least one vibration element 110 configured to vibrate upon receiving an activation signal, wherein the at least one vibration element 110 is configured to be in contact with a user's nose. More specifically the at least one vibration element 110 may be configured to be in contact with a part of the user's nose adjacent to a user's tear duct and/or external nasal nerve.

In embodiments, the wearable device 100 may be an eyewear, more specifically a pair of glasses, and in particular a pair of smart glasses.

In embodiments, the wearable device may comprise two vibration elements 110, in particular vibration plates or membranes, wherein at least one of the two vibration elements may be configured to be in contact with a nasal wing, in particular a part of the nasal wing adjacent to the user's tear duct and/or external nasal nerve.

In embodiments, the wearable device 100 may comprise a pair of lenses 130a, 130b disposed within a frame 140, wherein the frame 140 may comprise a bridge 150, in particular a bridge 150 disposed between the pair of lenses 130a, 130b.

In embodiments, the at least one vibration element 110 may be disposed within the frame 140, more specifically between the pair of lenses 130a, 130b and in particular within and/or adjacent to the bridge 150.

In embodiments, the wearable device 100 may comprise a sensor 160, wherein the sensor 160 may be configured to output the activation signal to the at least one vibration element 110 when the sensor 160 is activated.

In embodiments, the sensor 160 may be configured to measure one or more physiological parameters of the user, in particular one or more physiological parameters of an eye of the user.

In embodiments, the sensor 160 may be configured to measure a fluid's film thickness, more specifically a tear-film thickness, and in particular a tear-film thickness on an eye.

In embodiments, the sensor 160 may measure the fluid's film thickness by measuring changes in a reflectivity of the fluid film.

In embodiments, the sensor 160 may be configured to detect the user blinking, more specifically wherein the sensor 160 may be configured to detect a blinking rate of the user.

In embodiments, the sensor 160 may be configured to detect a user's eye color, more specifically a color of the user's sclera and in particular a degree of redness of the user's sclera.

In embodiments, the sensor 160 may comprise an optical sensor, in particular a reflectance sensor, light intensity sensor, spectrum sensor and/or color sensor.

In embodiments, the sensor 160 may comprise a camera.

In embodiments, the sensor 160 may be configured to measure the relative humidity and/or temperature of ambient air.

In embodiments, the sensor 160 may have an activation threshold value, wherein the sensor 160 may be configured to send an activation signal to the at least one vibration element 110 when a value detected by the sensor exceeds or falls below the threshold value.

In embodiments, the threshold value can be adjusted by the user.

In embodiments, the threshold value may be determined by a computer-implemented method.

In embodiments, the threshold value may be determined based on the user's medical data.

In embodiments, the wearable device 100 may comprise a fluid collector, more specifically wherein the fluid collector may be configured to collect tears excreted by the user, and in particular wherein the fluid collector may be configured to collect tears excreted by the user upon activation of at least one vibration element.

In embodiments, the wearable device 100 may comprise a tear analyzer, in particular a tear analyzer configured to analyze a chemical composition of tears collected by the fluid collector.

In embodiments, the wearable device 100 may comprise an actuation mechanism 170, configured to send the activation signal to the at least one vibration element 110 upon actuation.

In embodiments, the wearable device 100 may comprise a power source, in particular a rechargeable battery.

In embodiments, the at least one vibration element 110 may comprise a piezo-electric actuator, an eccentric rotating mass and/or a linear resonant actuator.

In embodiments, the at least one vibration element 110 may be configured to vibrate with a frequency between about 50 Hz to 1000 Hz, more specifically between about 150 Hz to about 500 Hz and in particular between about 200 Hz to about 300 Hz.

In embodiments, the at least one vibration element 110 may be configured to vibrate with an amplitude between about 0.05 mm to about 3 mm, more specifically, between about 0.1 mm to about 1.5 mm and in particular between about 0.9 mm to about 1.1 mm.

In embodiments, the wearable device 100 may comprise a processor 120, in particular a processor 120 configured to execute a computer-implemented method.

In a second aspect, the present disclosure relates to a computer-implemented method 1100. The computer-implemented method 1100 may comprise the following steps:
- receiving 1200 one or more physiological parameters of the user's eye from a sensor 160,
- comparing 1300 the one or more physiological parameters to one or more threshold values,
- outputting 1400 an activation signal to at least one vibration element 110 when:
   a) the one or more of the one or more physiological parameters exceeds one or more of the threshold values, or
   b) the one or more of the one or more physiological parameters falls below one or more of the threshold values.

In embodiments, the computer-implemented method may comprise receiving 1500 one or more environmental parameters from the sensor 160 and determining 1600 a presumed physiological state of the user's eye based on the one or more environmental parameters and in some examples the one or more physiological parameters. Additionally, the computer-implemented method may comprise a step of outputting 1400 the activation signal to the at least one vibration element 110 when the determined 1600 presumed physiological state deviates from a desired physiological state of the user's eye.

In embodiments, the computer-implemented method 1100 may comprise receiving 1700 an actuation signal from an actuation mechanism 170, wherein the actuation mechanism 170 is configured to send the actuation signal upon actuation. Additionally, the computer-implemented method 110 may comprise outputting 1400 an activation signal to at least one vibration element 110.

In embodiments, receiving 1200 the one or more physiological parameters may comprise receiving 1210 one or more images of the user's eye from the sensor 160 and determining 1220 one or more reflectivity's of the user's eye from the one or more images, wherein the one or more reflectivity's correspond to the one or more physiological parameters.

In embodiments, determining 1220 the one or more reflectivity's may comprise applying a fuzzy c means algorithm and/or generating a deep segmented output.

### Brief Description of the Drawings

Other characteristics will be apparent from the accompanying drawings, which form a part of this disclosure. The drawings are intended to further explain the present disclosure and to enable a person skilled in the art to practice it. However, the drawings are intended as non-limiting examples. Common reference numerals on different Figures indicate like or similar features.
**Fig. 1** is a perspective view a wearable device 100 according to the present disclosure.
**Figs. 2** schematically illustrates a computer-implemented method for activating a vibration element 110 based on one or more physiological parameters.
**Fig. 3** schematically illustrates a computer-implemented method for receiving one or more physiological parameters.

### Detailed Description

Hereinafter, a detailed description will be given of the present disclosure. The terms or words used in the description and the aspects of the present disclosure are not to be construed limitedly as only having common-language or dictionary meanings and should, unless specifically defined otherwise in the following description, be interpreted as having their ordinary technical meaning as established in the relevant technical field. The detailed description will refer to specific embodiments to better illustrate the present disclosure, however, it should be understood that the presented disclosure is not limited to these specific embodiments.

It has been found that the stimulation of a person's nose may lead to the expulsion of tears by the person's tear ducts. In particular, applying vibration to certain parts of the human nose may lead to expulsion of tears. However, carrying around a dedicated device to stimulate the nose may be tedious. Further, people may prefer to be observed stimulating their nose.

Accordingly, in a first aspect, the present disclosure relates to a wearable device 100 comprising at least one vibration element 110 configured to vibrate upon receiving an activation signal, wherein the at least one vibration element 110 is configured to be in contact with a user's nose.

The at least one vibration element 110 may stimulate the user's nose when vibrating. The vibration may lead to the expulsion of tears by the user's tear ducts. The expulsion of tears by the user may temporarily reduce or alleviate the symptoms of the dry eye syndrome. In particular, the expulsion of tears by the user due to the nose stimulation may reduce or alleviate the symptoms of the dry eye syndrome without requiring invasive surgery or the use of medication, such as eye drops, whose use may be uncomfortable and time-consuming.

It should be noted that the sensor 160 does not have to send the activation signal to the at least one vibration element 110 directly. For example, the sensor 160 may send a sensor signal to a processor 120, wherein the sensor signal is processed, and the activation signal may then be sent to the at least one vibration element 110 based on the sensor signal.

The term "configured to be in contact with a user's nose" within this disclosure may refer to the at least one vibration element 110 being in contact with the user's nose when the user is wearing the wearable device 100, in particular wearing it with a secure fit. The term "secure fit" within this disclosure may refer to the wearable device 100 staying in contact with a user's nose as long as no external force is applied. Additionally or alternatively, the term "secure fit" within this disclosure may refer to the wearable device staying in contact with the user's nose during regular motions performed by the user. Additionally or alternatively, the term "configured to be in contact with a user's nose" within this disclosure may refer to the at least one vibration element 110 being in contact with the user's nose when the user is wearing the wearable device 100 and wherein the wearable device 100 is attached to the user by one or more form, friction or force fits. The wearable device 100 may for example be attached to the user by one or more nose pads 180a, 180b and/or one or more temple tips 210 and/or temples 200. The one or more nose pads 180a, 180b may for example press against the user's nose creating a force fit. The one or more temple tips 210 may be disposed behind the user's ears leading to a form fit and/or the temples 200 may press against the user's skull for a force fit.

The wearable device 100 may be a wearable, also referred to as wearable technology, tech togs, smartwear, skin electronics. The term "wearable" within this disclosure may refer to electronic devices intended to be worn close or on the surface of the skin, wherein the electronic device may be configured to detect, analyze and/or transmit information concerning physiological parameters and/or ambient conditions. Additionally or alternatively, the term "wearable" within this disclosure may refer to electronic devices intended to be worn close or on the surface of the skin, wherein the electronic device is configured to stimulate part of the user's body. Additionally or alternatively, the term "wearable" within this disclosure may refer to electronic devices intended to be worn close or on the surface of the skin, wherein the electronic device may be configured to detect, analyze and/or transmit information concerning physiological parameters and/or ambient conditions, wherein the electronic device is further configured to stimulate part of the user's body, in particular the nose, based on the physiological parameters and/or ambient conditions.

More specifically the at least one vibration element 110 may be configured to be in contact with a part of the user's nose adjacent to a user's tear duct and/or external nasal nerve. The at least one vibration element 110 may stimulate the user's tear duct and/or external nasal nerve when in contact with a part of the user's nose adjacent to these structures. The stimulation of the user's tear ducts and/or external nasal nerve may efficiently and consistently lead to the expulsion of tears by the user.

In embodiments, the wearable device 100 may be an eyewear, more specifically a pair of glasses, and in particular a pair of smart glasses. The user may not be required to carry around a dedicated device, if the wearable device 100 is an eyewear. This may also reduce the likelihood other people notice that the user is using the wearable device to treat dry eye syndrome. Further, some eyewear, such as protection goggles, may be used in extreme ambient conditions, such as high heat, where dry eyes may occur more frequently. Additionally, many people wear a pair of glasses when looking at computer screens or while reading, e.g. during work. Looking at computer screens and/or reading may also increase the occurrence of dry eyes.

Smart glasses may already include a power source, e.g. a secondary battery, which may be used to power the at least one vibration element 110. Further, smart glasses may comprise sensors configured to measure ambient conditions or perform eye-tracking of the user.

In embodiments, the wearable device may comprise two vibration elements 110, in particular vibration plates or membranes, wherein at least one, in particular both of the two vibration elements, may be configured to be in contact with a nasal wing, in particular a part of the nasal wing adjacent to the user's tear duct and/or external nasal nerve.

In embodiments, the wearable device 100 may comprise a pair of lenses 130a, 130b disposed within a frame 140, wherein the frame 140 may comprise a bridge 150, in particular a bridge 150 disposed between the pair of lenses 130a, 130b.

In embodiments, the at least one vibration element 110 may be disposed within the frame 140, more specifically between the pair of lenses 130a, 130b and in particular within and/or adjacent to the bridge 150. The bridge 150 or components adjacent to the bridge may be in contact with the user's nose, more specifically in contact with a nasal wing, in particular a part of the nasal wing adjacent to the user's tear duct and/or external nasal nerve.

In embodiments, the wearable device 100 may comprise a sensor 160, wherein the sensor 160 may be configured to output the activation signal to the at least one vibration element 110 when the sensor 160 is activated.

The wearable device 100 may comprise a one or more nose pads 180a, 180b, in particular a pair of nose pads 180a, 180b. The nose pad(s) 180a, 180b may be configured to be in contact with a nasal wing, in particular a part of the nasal wing adj acent to the user's tear duct and/or external nasal nerve. Further, the nose pads may be configured to form a friction, force or form fit with the user's nose. In an example, the nose pads 180a, 180b may comprise or be attached to the at least one vibration element 110. The nose pads 180a, 180b may be disposed adjacent to the bridge 150 and/or frame 140 or may be integrally formed with the frame 140.

In embodiments, the nose pads 180a, 180b may be attached to the frame 140 and/or bridge 150 by one or more pad arms (not shown). The one or more pad arms may be configured to reduce a transfer of vibration from the at least one vibration element 110 and/or the one or more nose pads 180a, 180b to the frame 140. The one or more pad arms may comprise one or more vibration-damping elements. The vibration-damping element may be for example an elastomer, a foam, a gel, a spring, and/or a hydraulic system. The vibrations caused by the at least one vibration element 110 may be uncomfortable for the user. Reducing the vibration transferred to the frame 140 may hence increase the user comfort.

In embodiments, the sensor 160 may be configured to measure one or more physiological parameters of the user, in particular one or more physiological parameters of an eye of the user. The sensor 160 may comprise a plurality of sensors. For example, the sensor 160 may comprise a humidity sensor configured to measure the ambient air humidity and a temperature sensor configured to measure the temperature of the ambient air or the user. The sensor 160 may be configured to measure the relative humidity and/or temperature of ambient air.

Further, the sensor 160 may also comprise optical instruments, for example one or more cameras. The one or more cameras may record images, such as a images of visible light, infrared light and/or UV-light.

In embodiments, the sensor 160 may comprise a light source configured to emit a light beam and a reflectometer, in particular a spectrophotometer. The light source may illuminate the eye and the reflectometer may record a wavelength dependent reflection curve. The wavelength dependent reflection curve may be used to determine the film thickness. The sensor 160 may comprise a galvanometer scanner configured to guide the light beam emitted by the light source.

In embodiments, the sensor 160 may comprise an optical sensor, in particular a reflectance sensor, light intensity sensor, spectrum sensor and/or color sensor.

In embodiments, the sensor 160 may be configured to measure a fluid's film thickness, more specifically a tear-film thickness, and in particular a tear-film thickness on an eye. The eye may be the user's eye. The thickness of the fluid film on the eye may indicate whether the eye is suffering from dryness. The fluid film may comprise other components than tear fluid, for example, if the user has used artificial tears.

In embodiments, the sensor 160 may measure the fluid's film thickness by measuring a reflectivity of the fluid film and/or changes in a reflectivity of the fluid film.

In embodiments, the sensor 160 may be configured to detect the user blinking, more specifically wherein the sensor 160 may be configured to detect a blinking rate of the user. The blinking rate of the user may be indicative of dry eyes, in particular an increased blinking rate of the user may indicate dry eyes.

In embodiments, the sensor 160 may be configured to detect a user's eye color, more specifically a color of the user's sclera and in particular a degree of redness of the user's sclera. A change in the user's eye color, in particular the user's sclera may be indicative of dry eyes. Dryness of the eyes may cause irritation of the eyes, which may lead to injection and prominence of the superficial blood vessels of the conjunctiva. The conjunctiva within this disclosure may be considered part of the sclera. The conjunctiva covers the sclera. Hence, measuring a color change of the of the user's sclera, in particular an increase in redness, may indicate dry eyes.

In embodiments, the sensor 160 may have an activation threshold value, wherein the sensor 160 is configured to send an activation signal to the at least one vibration element 110 when a value detected by the sensor exceeds or falls below the threshold value. For example, the sensor 160 may be configured to send the activation signal when the redness and/or ambient threshold of the exceeds a threshold. Alternatively or additionally, the sensor 160 may be configured to send the activation signal to the at least one vibration element 110 when the fluid film thickness falls below a threshold. The sensor 160 may be configured to send the activation signal when multiple threshold values detected by the sensor fall below or exceed a threshold. The threshold may be pre-set, for example based on medical data, e.g. a physiological fluid film thickness of the user. The value detected by the sensor may be for example a physiological parameter of the user, for example the tear-film thickness on the user's eye, the blinking rate, the ambient air temperature and/or ambient air humidity. Alternatively or additionally, in embodiments, the sensor 160 may send a sensor signal to the processor 120. The processor 120 may then determine whether the sensor signal indicates that the one more physiological parameters and/or ambient conditions exceed or fall below the activation threshold value. The processor 120 may then send the activation signal to the at least one vibration element 110 depending on an outcome of the determination.

In embodiments, the threshold value can be adjusted by the user. Some users may prefer the at least one vibration element 110 to vibrate at a higher or lower fluid film thickness. In embodiments, the threshold value may be determined based on the user's medical data.

In embodiments, the threshold value may be determined by a computer-implemented method. In embodiments, the wearable device 100 may comprise the processor 120. The processor 120 configured to execute a computer-implemented method. For example, the computer-implemented method may take into account ambient conditions, such as temperature and/or humidity, and user preferences and/or medical data to compute user specific threshold values.

In embodiments, the wearable device 100 may comprise a fluid collector, more specifically wherein the fluid collector may be configured to collect tears excreted by the user, and in particular wherein the fluid collector may be configured to collect tears excreted by the user upon activation of at least one vibration element. Tear fluid may be used for analysis, in particular medical analysis. The composition of tear fluid and changes in the composition of tear fluid may allow drawing conclusions on the user's health. The presence of certain compounds may be for example indicative of certain illness, such as Alzheimer's. The wearable device 100 may therefore be used to collect tears with reduced discomfort to the user compared to other methods. Further, the tear collector may collect tears during regular use. The collected fluid, more specifically the tears excreted by the user, may then be analyzed within a lab or with analysis device disposed within the wearable device 100. The tear collector may also comprise a dispensing element configured to dispense the collected tears. The tears may for example be used for a lateral flow test to detect the presence of a target substance, such as a specific protein.

In embodiments, the wearable device 100 may comprise a tear analyzer, in particular a tear analyzer configured to analyze a chemical composition of tears collected by the fluid collector. Analysis devices, in particular lab-on-a-chip devices may be integrated into the wearable device 100 for analysis.

In embodiments, the wearable device 100 may comprise an actuation mechanism 170, configured to send the activation signal to the at least one vibration element 110 upon actuation of the actuation mechanism. The actuation mechanism 170 may for example comprise a button, which the user can push to activate the at least one vibration element 110, if the user feels that his eyes are dry. The actuation mechanism 170 may also comprise a motion detection sensor. For example, the user may move his hand in front of the actuation mechanism 170 to actuate it. The actuation mechanism 170 may also comprise an actuation mechanism camera. The actuation mechanism camera may record images or videos. Based on the recorded images or videos specific hand gestures may be identified, upon whose recognition the actuation mechanism 170 may be actuated. For example, move two fingers from the frame 140 towards the temple tip 210, which may be recorded by the actuation mechanism camera. The records by the actuation mechanism camera may then be processed in the processor 120 and used to actuate the actuation mechanism 170.

It should be noted that the actuation mechanism 170 does not have to send the activation signal to the at least one vibration element 110 directly. For example, the actuation mechanism 170 may send an actuation signal to the processor 120, wherein the actuation signal is processed, and the activation signal may then be sent to the at least one vibration element 110 based on the actuation signal. Further, the actuation signal may also take the form of an electrical current sufficient to power the at least one vibration element 110. For example, actuation mechanism 170 may comprise part of an electric circuit, more specifically an electric switch, wherein the at least one vibration element is also part of the electric circuit. Upon actuation of the actuation mechanism 170 the electric circuit is closed and the at least one vibration element supplied with the electric circuit.

In embodiments, the wearable device 100 may comprise a power source, in particular a rechargeable battery.

In embodiments, the at least one vibration element 110 may comprise a piezo-electric actuator, an eccentric rotating mass and/or a linear resonant actuator. The piezo-electric actuator may be for example a piezo-electric crystal, wherein the piezo-electric crystal is attached to a plate or a membrane in contact with the user's nose. Similarly, the eccentric rotating mass or linear resonant actuator may also be attached to a plate or membrane in contact with the user's nose. The piezo-electric actuator may also be a foil. For example, polyvinylidene fluoride may form a foil and may be piezoelectric.

In embodiments, the at least one vibration element 110 may be configured to vibrate with a frequency between about 50 Hz to 1000 Hz, more specifically between about 150 Hz to about 500 Hz and in particular between about 200 Hz to about 300 Hz. Further, the at least one vibration element 110 may be configured to vibrate with an amplitude between about 0.05 mm to about 3 mm, more specifically, between about 0.1 mm to about 1.5 mm and in particular between about 0.9 mm to about 1.1 mm. The aforementioned frequency and/or amplitude may be effective in leading to tear expulsion by the user.

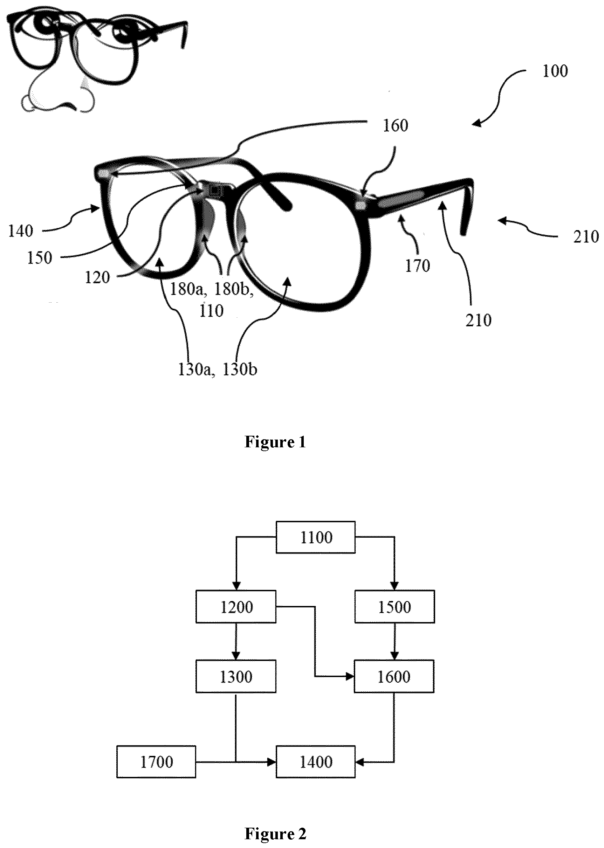

Figure 1 shows an embodiment of the wearable device 100 according to the present disclosure. Figure 1 comprises two vibration elements 110 disposed within or attached to two nose pads 180a, 180b. When the wearable device 100 is worn the two nose pads 180a, 180b are each in contact with a nasal wing of the user. The wearable device 100 further comprises two sensors 160. The two sensors 160 are depicted as facing forwards. The sensor 160 may also face inwards, more specifically the sensor 160 may face towards the user's eyes. In an example, the wearable device depicted in Figure 1 comprises an actuation mechanism 170 disposed within one of the temples 200 and temple tips 210. In an example, the wearable device 100 of Figure 1 may comprise the pair of lenses 130a, 130b which are disposed within the frame 140.

In a second aspect, the present disclosure relates to a computer-implemented method 1100. The computer-implemented method 1100 may comprise the following steps, which are also depicted in Figure 2:
- receiving 1200 one or more physiological parameters of the user's eye from the sensor 160,
- comparing 1300 the one or more physiological parameters to one or more threshold values,
- outputting 1400 the activation signal to the at least one vibration element 110 when:
   a) the one or more of the one or more physiological parameters exceeds one or more of the threshold values, or
   b) the one or more of the one or more physiological parameters fall below one or more of the threshold values.

The one or more physiological parameters may be for example a tear-film thickness on a user's eye or the redness of a user's eye. If the tear-film thickness falls below a certain threshold value or the redness of the user's eye exceeds a certain threshold value the computer-implemented may send the activation signal to the at least one vibration element 110. For example, if the at least one vibration element 110 is disposed within a wearable device 100 as described above, the wearable device's 100 at least one vibration element 110 may start vibrating, which may cause the user to expulse tears, reducing the dryness of the user's eye the tear is expulsed into.

In embodiments, the computer-implemented method 1100 may comprise receiving 1500 one or more environmental parameters from the sensor 160 and determining 1600 a presumed physiological state of the user's eye based on the one or more environmental parameters and, in examples, the one or more physiological parameters. Additionally, the computer-implemented method may comprise a step of outputting 1400 the activation signal to the at least one vibration element 110 when the determined 1600 presumed physiological state deviates from a desired physiological state of the user's eye. For example, the sensor 160 may measure a high temperature and low air humidity. Based on this the presumed physiological state may be determined 1600 as "dry eye". The desired physiological state of the user's eye may be "sufficiently wet". As a result, the activation signal may be output 1400. The presumed physiological state of the user's eye may also be determined 1600 by a combination of the one or more environmental parameters together with the one or more physiological parameters.

In embodiments, the computer-implemented method 1100 may comprise receiving 1700 an actuation signal from the actuation mechanism 170, wherein the actuation mechanism 170 is configured to send the actuation signal upon actuation. In an example, the computer-implemented method 110 may comprise outputting 1400 an activation signal to the at least one vibration element 110.

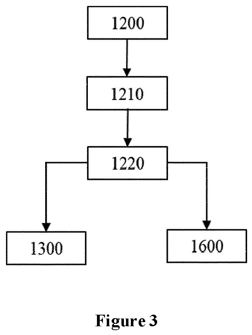

In embodiments, as depicted in Figure 3, receiving 1200 the one or more physiological parameters may comprise receiving 1210 one or more images of the user's eye from the sensor 160 and determining 1220 one or more reflectivity's of the user's eye from the one or more images, wherein the one or more reflectivity's correspond to the one or more physiological parameters.

In embodiments, determining 1220 the one or more reflectivity's may comprise applying a fuzzy c means algorithm and/or generating a deep segmented output. The fuzzy c means algorithm and/or the generation of the deep segmented output may allow identification of the one or more physiological parameters of the user's eye with high accuracy.

The computer-implemented method 1100 may also only be partly executed on the processor 120. The processor 120 may comprise an interface with a second processor. The second processor may be for example within a mobile device, such as a smart phone. This may allow reducing the required size of the processor 120, which may make it easier to accommodate the processor 120 within the wearable device 100 and reduce its power consumption. Further, mobile device may comprise a user interface, for example an app. The user may be able to apply changes to the wearable device 100 through the app, for example by setting or adjusting the one or more threshold values.

Although the present disclosure is defined in the attached claims, it should be understood that the present disclosure can also (alternatively) be defined in accordance with the following aspects:
1. In a first aspect, the present disclosure relates to a wearable device (100) comprising at least one vibration element (110) configured to vibrate upon receiving an activation signal, wherein the at least one vibration element (110) is configured to be in contact with a user's nose, more specifically a part of the user's nose adj acent to a user's tear duct and/or external nasal nerve.
2. The wearable device (100) according to aspect 1, wherein the wearable device (100) is an eyewear, more specifically a pair of glasses, and in particular a pair of smart glasses.
3. The wearable device (100) according to any preceding aspect, wherein the wearable device (100) comprises two vibration elements (110), in particular vibration plates or membranes, wherein at least one of the two vibration elements is configured to be in contact with a nasal wing, in particular a part of the nasal wing adjacent to the user's tear duct and/or external nasal nerve.
4. The wearable device (100) according to any preceding aspect, wherein the wearable device (100) comprises a pair of lenses (130a, 130b) disposed within a frame (140), wherein the frame (140) comprises a bridge (150), in particular a bridge (150) disposed between the pair of lenses (130a, 130b).
5. The wearable device (100) according to aspect 4, wherein the at least one vibration element (110) is disposed within the frame (140), more specifically between the pair of lenses (130a, 130b) and in particular within and/or adjacent to the bridge (150).
6. The wearable device (100) according to any preceding aspect, wherein the wearable device (100) comprises a sensor (160), wherein the sensor (160) is configured to output the activation signal to the at least one vibration element (110) when the sensor (160) is activated.
7. The wearable device (100) according to aspect 6, wherein the sensor (160) is configured to measure one or more physiological parameters of the user, in particular one or more physiological parameters of an eye of the user.
8. The wearable device (100) according to aspect 6 or 7, wherein the sensor (160) is configured to measure a fluid's film thickness, more specifically a tear-film thickness, and in particular a tear-film thickness on an eye.
9. The wearable device (100) according to aspect 8, wherein the sensor (160) measures the fluid's film thickness by measuring changes in a reflectivity of the fluid film.
10. The wearable device (100) according to any of aspects 6 to 9, wherein the sensor (160) is configured to detect the user blinking, more specifically wherein the sensor (160) is configured to detect a blinking rate of the user.
11. The wearable device (100) according to any of aspects 6 to 10, wherein the sensor (160) is configured to detect a user's eye color, more specifically a color of the user's sclera and in particular a degree of redness of the user's sclera.
12. The wearable device (100) according to any of aspects 6 to 11, wherein the sensor (160) comprises an optical sensor, in particular a reflectance sensor, light intensity sensor, spectrum sensor and/or color sensor.
13. The wearable device (100) according to any of aspects 6 to 12, wherein the sensor (160) comprises a camera.
14. The wearable device (100) according to any of aspects 6 to 13, wherein the sensor (160) is configured to measure the relative humidity and/or temperature of ambient air.
15. The wearable device (100) according to any of aspects 6 to 14, wherein the sensor (160) has an activation threshold value, wherein the sensor (160) is configured to send an activation signal to the at least one vibration element (110) when a value detected by the sensor exceeds or falls below the threshold value.
16. The wearable device (100) according to aspect 15, wherein the threshold value can be adjusted by the user.
17. The wearable device (100) according to aspect 15 or 16, wherein the threshold value is determined by a computer-implemented method.
18. The wearable device (100) according to any one of aspects 15 to 17, wherein the threshold value is determined based on the user's medical data.
19. The wearable device (100) according to any preceding aspect, wherein the wearable device (100) comprises a fluid collector, more specifically wherein the fluid collector is configured to collect tears excreted by the user, and in particular wherein the fluid collector is configured to collect tears excreted by the user upon activation of at least one vibration element.
20. The wearable device (100) according to any preceding aspect, in particular aspect 19, wherein the wearable device (100) comprises a tear analyzer, in particular a tear analyzer configured to analyze a chemical composition of tears collected by the fluid collector.
21. The wearable device (100) according to any preceding aspect, wherein the wearable device (100) comprises an actuation mechanism (170), configured to send the activation signal to the at least one vibration element (110) upon actuation.
22. The wearable device (100) according to any preceding aspect, wherein the wearable device (100) comprises a power source, in particular a rechargeable battery.
23. The wearable device (100) according to any preceding aspect, wherein the at least one vibration element (110) comprises a piezo-electric actuator, an eccentric rotating mass and/or a linear resonant actuator.
24. The wearable device (100) according to any preceding aspect, wherein the at least one vibration element (110) is configured to vibrate with a frequency between about 50 Hz to 1000 Hz, more specifically between about 150 Hz to about 500 Hz and in particular between about 200 Hz to about 300 Hz.
25. The wearable device (100) according to any preceding aspect, wherein the at least one vibration element (110) is configured to vibrate with an amplitude between about 0.05 mm to about 3 mm, more specifically, between about 0.1 mm to about 1.5 mm and in particular between about 0.9 mm to about 1.1 mm.
26. The wearable device (100) according to any preceding aspect, wherein the wearable device (100) comprises a processor 120, in particular a processor 120 configured to execute a computer-implemented method.
27. A computer-implemented method (1100) comprising:
   - receiving (1200) one or more physiological parameters of the user's eye from a sensor (160),
   - comparing (1300) the one or more physiological parameters to one or more threshold values,
   - outputting (1400) an activation signal to at least one vibration element (110) when:
      a) the one or more of the one or more physiological parameters exceeds one or more of the threshold values, or
      b) the one or more of the one or more physiological parameters falls below one or more of the threshold values.
28. The computer-implemented method (1100) according to aspect 27, wherein the computer-implemented method comprises:
   - receiving (1500) one or more environmental parameters from the sensor (160),
   - determining (1600) a presumed physiological state of the user's eye based on the one or more environmental parameters and, in examples, the one or more physiological parameters;
   outputting (1400) the activation signal to the at least one vibration element (110) when the determined (1600) presumed physiological state deviates from a desired physiological state of the user's eye.
29. The computer-implemented method (1100) according to aspect 27 or 28, wherein the computer-implemented method (1100) comprises:
   - receiving (1700) an actuation signal from an actuation mechanism (170), wherein the actuation mechanism (170) is configured to send the actuation signal upon actuation;
   - outputting (1400) an activation signal to at least one vibration element (110).
30. The computer-implemented method (1100) according to one of aspects 27 to 29, wherein receiving (1200) the one or more physiological parameters comprises:
   - receiving (1210) one or more images of the user's eye from the sensor (160)
   - determining (1220) one or more reflectivity's of the user's eye from the one or more images, wherein the one or more reflectivity's correspond to the one or more physiological parameters.
31. The computer-implemented method (1100) according to aspect 30, wherein determining (1220) the one or more reflectivity's comprises applying a fuzzy c means algorithm and/or generating a deep segmented output.

## Claims

1. A wearable device (100) comprising at least one vibration element (110) configured to vibrate upon receiving an activation signal,
wherein the at least one vibration element (110) is configured to be in contact with a user's nose.

2. The wearable device (100) according to claim 1, wherein the wearable device (100) is an eyewear.

3. The wearable device (100) according to any preceding claim, wherein the wearable device (100) comprises two vibration elements (110).

4. The wearable device (100) according to any preceding claim, wherein the wearable device (100) comprises a pair of lenses (130a, 130b) disposed within a frame (140), wherein the frame (140) comprises a bridge (150).

5. The wearable device (100) according to claim 4, wherein the at least one vibration element (110) is disposed within the frame (140).

6. The wearable device (100) according to any preceding claim, wherein the wearable device (100) comprises a sensor (160), wherein the sensor (160) is configured to output the activation signal to the at least one vibration element (110) when the sensor (160) is activated.

7. The wearable device (100) according to claim 6, wherein the sensor (160) is configured to measure one or more physiological parameters of the user.

8. The wearable device (100) according to claim 6 or 7, wherein the sensor (160) is configured to measure a fluid's film thickness, more specifically a tear-film thickness, and in particular a tear-film thickness on an eye.

9. The wearable device (100) according to claim 8, wherein the sensor (160) measures the fluid's film thickness by measuring changes in a reflectivity of the fluid film.

10. The wearable device (100) according to any of claims 6 to 9, wherein the sensor (160) is configured to detect the user blinking.

11. The wearable device (100) according to any of claims 6 to 10, wherein the sensor (160) is configured to detect a user's eye color, more specifically a color of the user's sclera and in particular a degree of redness of the user's sclera.

12. The wearable device (100) according to any of claims 6 to 11, wherein the sensor (160) comprises an optical sensor.

13. The wearable device (100) according to any of claims 6 to 12, wherein the sensor (160) has an activation threshold value, wherein the sensor (160) is configured to send an activation signal to the at least one vibration element (110) when a value detected by the sensor exceeds or falls below the threshold value.

14. The wearable device (100) according to any preceding claim, wherein the wearable device (100) comprises a fluid collector, wherein the fluid collector is configured to collect tears excreted by the user, and wherein the fluid collector is configured to collect tears excreted by the user upon activation of at least one vibration element.

15. The wearable device (100) according to claim 14, wherein the wearable device (100) comprises a tear analyzer, configured to analyze a chemical composition of tears collected by the fluid collector.
